# EUROPEAN PATENT SPECIFICATION

(11) **EP 4 376 922 B1**
(45) Date of publication and mention of the grant of the patent: **02.09.2026**
(21) Application number: 22754926.8
(22) Date of filing: 28.07.2022
(51) Int. Cl.: A61M 5/31, A61M 5/315

(54) **SYRINGE GRIP ASSEMBLY**
SPRITZENGRIFFBAUGRUPPE
ASSEMBLAGE DE LA POIGNÉE DE LA SERINGUE

(30) Priority: 30.07.2021 US 202163227857 P
(43) Date of publication of application: 05.06.2024
(73) Proprietor: Galderma Holding SA, 6300 Zug (CH)
(72) Inventor: ULLSTEN, Oscar, 75330 Uppsala (SE); STRÅHLE, Sofie, 75335 Uppsala (SE)
(74) Representative: Impact Intellectual Property LLP
(86) International application number: PCT/IB2022/057027
(87) International publication number: WO 2023/007442

(56) References cited:
- US-A1- 2016 038 682
- US-A1- 2020 197 924

## Description

### FIELD

The present disclosure relates generally to the field of syringes.

### BACKGROUND

The following description of the background of the present technology is provided to aid in understanding the present technology. US 2016/038682 presents the relevant state of the art corresponding to the preamble of claim 1.

When a user of a syringe administers a substance, it is common, in particular within certain fields of treatment, to distribute the total volume carried by the syringe over a treatment area. Some examples of these fields of treatment are dermatology, plastic surgery, cosmetic surgery, and odontology. In these cases, the distribution may be done, for example, by inserting the needle of the syringe just beneath and approximately in parallel with the skin surface and then administering the substance. Another way is to administer the substance at several anatomic locations within a limited area. For example, the user may administer a fraction of the total volume carried by the syringe at each location. The accuracy of administering a substance in typical syringes is affected by various factors.

### SUMMARY

The invention is defined according to claim 1 and the dependent claims 2-14. Certain syringes are constructed so that, to allow the user to know the fraction of the total volume at each location, the syringe barrel is typically provided with a gradation scale (e.g., the gradation scale may be printed on a label and then attached to the syringe barrel), and the user observes the position of the plunger along the scale. Additionally, the syringe may sometimes be held at an angle where the scale is hidden. Further, another problem with the use of a gradation scale is the fact that the user has to observe it during the injection, which results in a distraction of attention arising when the user has to look at the gradation scale instead of focusing on the course of events at the treatment area. These and other issues affecting administration of a substance are addressed by the techniques of the present disclosure.

The invention refers to a syringe grip assembly configured to receive a syringe barrel. The syringe grip assembly comprises a plunger rod, a syringe grip, and an engagement member. The plunger rod has a ribbed outer surface. The syringe grip includes an activation button segment and a central opening. The central opening extends through the syringe grip and is configured to receive the plunger rod. The engagement member includes a rod engagement segment that is arranged at a non-normal angle (that is, an angle other than 90°) with respect to a central axis of the plunger rod. The engagement member is selectively moveable using the activation button segment between (i) an engaged position, in which the rod engagement segment is in contact with the plunger rod, and (ii) a disengaged position, in which the rod engagement segment is separated from the plunger rod.

In some embodiments, the ribbed outer surface includes a plurality of ribs and, when the engagement member is in the engaged position, the engagement member is configured to allow the plunger rod to move through the central opening in a first direction and, as the plunger rod moves in the first direction, the rod engagement segment is configured to sequentially snap over each rib of the plurality of ribs.

In some embodiments, the syringe grip is configured to provide at least one of tactile feedback or audible feedback to a user each time the rod engagement segment snaps over a rib of the plurality of ribs, the at least one of tactile feedback or audible feedback being indicative of an amount of substance being dispensed through the syringe barrel.

In some embodiments, the syringe grip includes a support lip adjacent to the central opening and configured to provide support to the plunger rod such that the plunger rod is maintained in axial alignment with the central opening.

In some embodiments, the activation button segment includes at least one grip protrusion on an external surface of the activation button segment.

According to the invention, the activation button segment is moveable between a lowered position, in which the engagement member is moved to the engaged position, and a raised position, in which the engagement member is moved to the disengaged position.

In some embodiments, the syringe grip includes a lower grip component and an upper grip component, the lower grip component including a lower gripping surface and a receiving section, the upper grip component received within the receiving section and including the activation button segment.

In some embodiments, the receiving section of the lower grip component includes an opening extending through a longitudinal end of the receiving section, and the activation button segment extends beyond the longitudinal end of the receiving section such that the activation button segment is accessible from a top surface and a bottom surface to allow for the activation button segment to be selectively moved between the raised position and the lowered position.

Another embodiment relates to a syringe grip assembly configured to receive a syringe barrel. The syringe grip assembly comprises a plunger rod, a syringe grip, and an engagement member. The plunger rod has a ribbed outer surface. The syringe grip includes a lower grip component, an upper grip component, and a central opening. The lower grip component includes a receiving section. The upper grip component is received within the receiving section and includes an activation button segment. The activation button segment includes at least one grip protrusion on an external surface of the activation button segment. The central opening extends through the syringe grip and is configured to receive the plunger rod. The engagement member includes a rod engagement segment. The engagement member is selectively moveable using the activation button segment between an engaged position, where the rod engagement segment is in contact with the plunger rod, and a disengaged position, where the rod engagement segment is separated from the plunger rod.

In some embodiments, the ribbed outer surface includes a plurality of ribs and, when the engagement member is in the engaged position, the engagement member is configured to allow the plunger rod to move through the central opening in a first direction and, as the plunger rod moves in the first direction, the rod engagement segment is configured to sequentially snap over each rib of the plurality of ribs.

In some embodiments, the syringe grip is configured to provide at least one of tactile feedback or audible feedback to a user each time the rod engagement segment snaps over a rib of the plurality of ribs, the at least one of tactile feedback or audible feedback being indicative of an amount of substance being dispensed through the syringe barrel.

In some embodiments, when the engagement member is in the engaged position, the engagement member is configured to prevent the plunger rod from moving through the central opening in a second direction, opposite the first direction.

In some embodiments, the rod engagement segment is arranged at a non-normal angle with respect to a central axis of the plunger rod.

In some embodiments, the upper grip component includes a support lip adjacent to the central opening and configured to provide support to the plunger rod such that the plunger rod is maintained in axial alignment with the central opening.

Another embodiment relates to a syringe grip assembly configured to receive a syringe barrel. The syringe grip assembly comprises a plunger rod, a syringe grip, and an engagement member. The plunger rod has a ribbed outer surface. The syringe grip includes an activation button segment, a central opening, and a support lip. The central opening extends through the syringe grip and is configured to receive the plunger rod. The support lip is adjacent to the central opening and is configured to provide support to the plunger rod such that the plunger rod is maintained in axial alignment with the central opening. The engagement member includes a rod engagement segment. The engagement member being selectively moveable using the activation button segment between an engaged position, where the rod engagement segment is in contact with the plunger rod, and a disengaged position, where the rod engagement segment is separated from the plunger rod.

In some embodiments, the rod engagement segment is arranged at a non-normal angle with respect to a central axis of the plunger rod.

In some embodiments, the syringe grip includes a lower gripping surface defining at least one concavity.

In some embodiments, the activation button segment includes at least one grip protrusion on an external surface of the activation button segment.

In some embodiments, the activation button segment is moveable between a lowered position, in which the engagement member is moved to the engaged position, and a raised position, in which the engagement member is moved to the disengaged position.

In some embodiments, the activation button segment is accessible from a top surface and a bottom surface to allow for the activation button segment to be selectively moved between the raised position and the lowered position.

### BRIEF DESCRIPTION OF THE DRAWINGS

Further features, characteristics, and advantages of the present disclosure will become apparent to a person of ordinary skill in the art from the following detailed description of embodiments of the present disclosure, made with reference to the drawings annexed, in which like reference characters refer to like elements.
FIG. 1 is a front, top, right perspective view of a syringe having a syringe grip, according to an example embodiment.
FIG. 2 is a rear, bottom, left perspective view of the syringe of FIG. 1, according to an example embodiment.
FIG. 3 is a front view of the syringe of FIG. 1, according to an example embodiment.
FIG. 4 is a left side view of the syringe of FIG. 1, according to an example embodiment.
FIG. 5 is a right side view of the syringe of FIG. 1, according to an example embodiment.
FIG. 6 is a rear view of the syringe of FIG. 1, according to an example embodiment.
FIG. 7 is a top view of the syringe of FIG. 1, according to an example embodiment.
FIG. 8 is a bottom view of the syringe of FIG. 1, according to an example embodiment.
FIG. 9 is a front view of a plunger of the syringe of FIG. 1, according to an example embodiment.
FIG. 10 is a front, top, right perspective view of a lower grip component of the syringe of FIG. 1, according to an example embodiment.
FIG. 11 is a rear view of the lower syringe grip of FIG. 10, according to an example embodiment.
FIG. 12 is a front, top, right perspective view of an upper grip component of the syringe of FIG. 1, according to an example embodiment.
FIG. 13 is a rear, bottom, left perspective view of the upper grip component of FIG. 12, according to an example embodiment.
FIG. 14 is a front view of an engagement member of the syringe of FIG. 1, according to an example embodiment.
FIG. 15 is a front, top, right perspective view of the engagement member of FIG. 14, according to an example embodiment.
FIG. 16 is a cross-section view of the syringe of FIG. 1, taken along line 16-16, according to an example embodiment.
FIG. 17 is a front, bottom, left perspective view of the upper grip component, showing the engagement member inserted therein, according to an example embodiment.
FIG 18 is a rear, top perspective view of the lower grip component, showing the engagement member inserted therein, according to an example embodiment.
FIG. 19 is a detail rear view of the syringe of FIG. 1, shown without the upper grip component and the outer sheath of the syringe, according to an example embodiment.
FIG. 20 is a front, top, right perspective view of another syringe grip assembly, according to an example embodiment.
FIG. 21 is a front view of the syringe grip assembly of FIG. 20, according to an example embodiment.
FIG. 22 is a bottom view of the syringe grip assembly of FIG. 20, according to an example embodiment.
FIG. 23 is a front, top, right perspective view of a lower grip component of the syringe grip assembly of FIG. 20, according to an example embodiment.
FIG. 24 is a top view of an upper grip component of the syringe grip assembly of FIG. 20, according to an example embodiment.
FIG. 25 is a rear, bottom, left perspective view of the upper grip component of FIG. 24, according to an example embodiment.

### DETAILED DESCRIPTION

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

The following terms are used throughout and are as defined below.

As used herein and in the appended claims, singular articles such as "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of refereeing individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the embodiments and does not pose a limitation on the scope of the claims unless otherwise stated. No language in the specification should be construed as indicating any non-claimed element as essential.

The embodiments illustratively described herein may suitably be practiced in the absence of any element or elements, limitation or limitations, not specifically disclosed herein. Thus, for example, the terms "comprising," "including," "containing," etc. shall be read expansively and without limitation. Additionally, the phrase "consisting essentially of" will be understood to include those elements specifically recited and those additional elements that do not materially affect the basic and novel characteristics of the claimed technology. The phrase "consisting of" excludes any element not specified. The expression "comprising" means "including, but not limited to." Thus, other non-mentioned substances, additives, carriers, or steps may be present. Unless otherwise specified, "a" or "an" means one or more.

Unless otherwise indicated, all numbers expressing quantities of properties, parameters, conditions, and so forth, used in the specification and claims are to be understood as being modified in all instances by the term "about." Accordingly, unless indicated to the contrary, the numerical parameters set forth in the following specification and attached claims are approximations. Any numerical parameter should at least be construed in light of the number reported significant digits and by applying ordinary rounding techniques. The term "about" when used before a numerical designation, e.g., temperature, time, amount, and concentration including range, indicates approximations which may vary by (+) or (-) 10%, 5% or 1%.

As will be understood by one of skill in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into subranges as discussed above. Finally, as will be understood by one skilled in the art, a range includes each individual member.

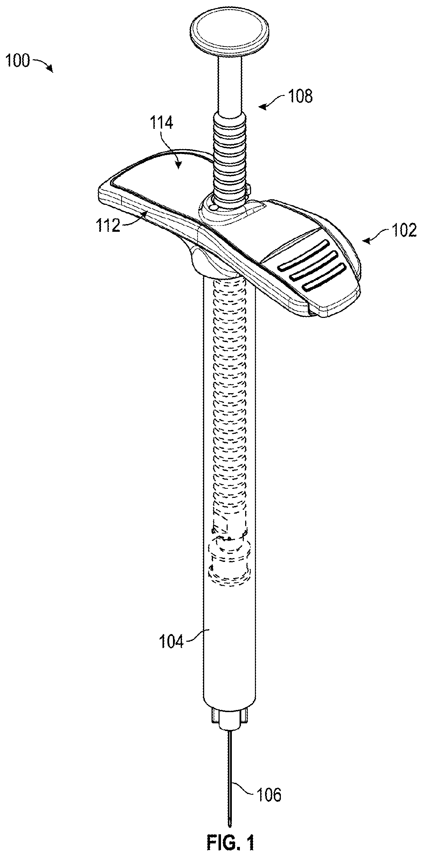

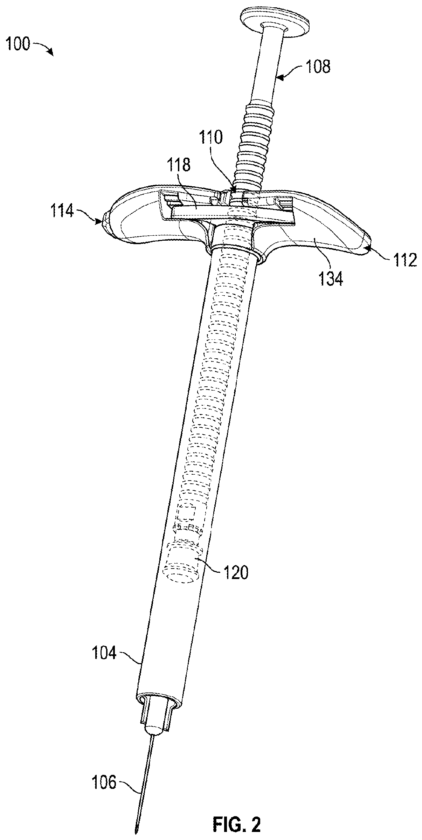

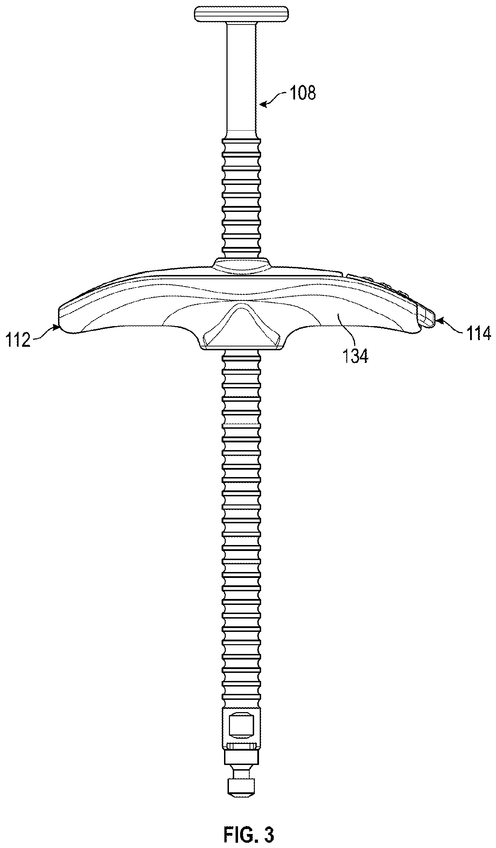

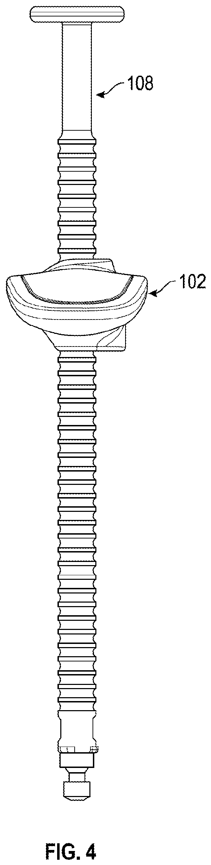

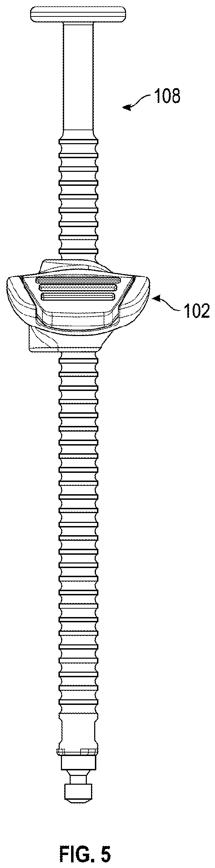

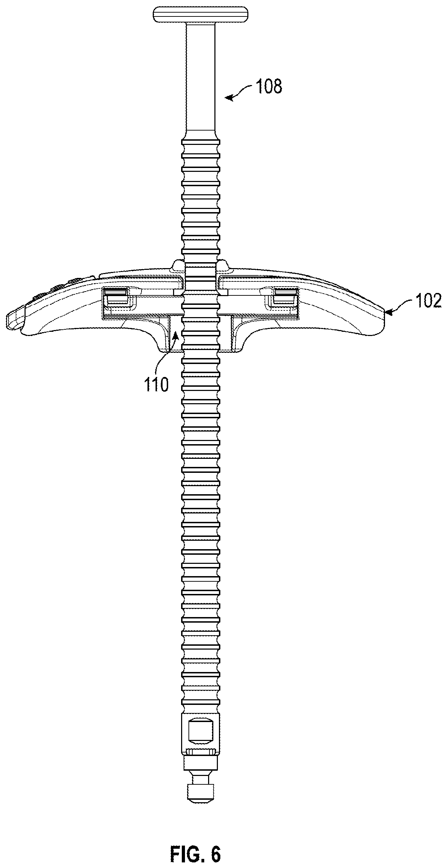

Referring now to FIGS. 1-8 generally, a syringe 100 having a syringe grip assembly 102 attached thereto is shown, according to an example embodiment. The syringe grip assembly 102 is generally configured to allow for a controlled amount of a substance to be dispensed from within a barrel 104 (shown in FIGS. 1 and 2) of the syringe 100, out through a needle 106 (shown in FIGS. 1 and 2) of the syringe 100, and injected into a patient or subject at a desired location.

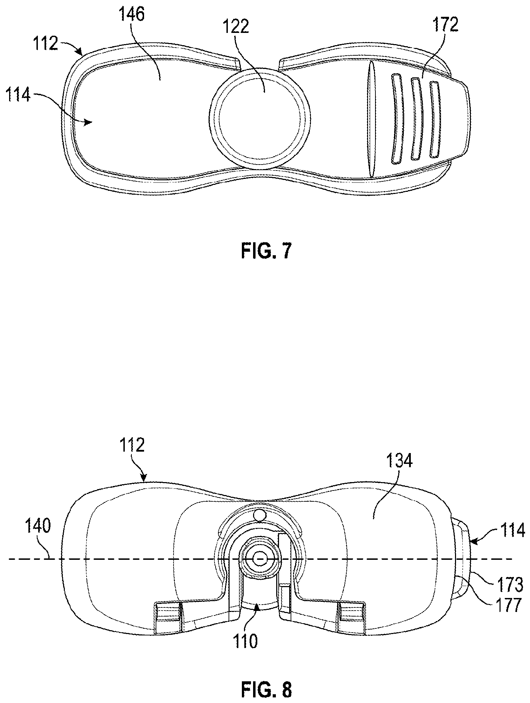

For example, the syringe grip assembly 102 includes a plunger rod 108 configured to be received (along with the barrel 104) within a central grip opening 110 (as shown in FIGS. 2 and 8) formed by a syringe grip formed by a lower grip component 112 and an upper grip component 114 of the syringe grip assembly 102. The plunger rod 108 is further configured to be inserted into a central bore of the barrel 104 through an aperture in the top of the barrel 104 (e.g., passing through a top surface of a barrel flange 118 shown in FIG. 2). When inserted into the central bore of the barrel 104, a plunger or seal 120 that is attached to the plunger rod 108 is configured to form a seal with an inner wall of the central bore at the distal end of the plunger rod 108 such that, as the plunger rod 108 is advanced into the central bore of the barrel 104, a substance disposed within the barrel 104 is forced out of the needle 106.

In some embodiments, the barrel 104 (and the barrel flange 118) may be formed of a plastic material or a glass material. The type of barrel 104 utilized may, for example, be determined based on the intended injection application, the static or sticking force that would be created between the plunger or seal 120 and the inner surface of the barrel 104, and differences in leakage force between glass and plastic barrels. As an example, in some instances, the barrel 104 may be that of a 0.5 ml glass syringe. In some scenarios, the plunger rod 108 may be sized to match or otherwise function with the barrel or syringe used.

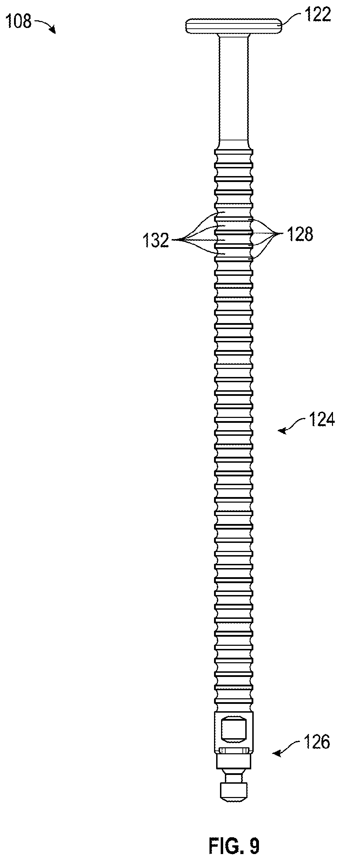

As best shown in FIG. 9, the plunger rod 108 includes a rod head 122, a ribbed portion 124, and a plunger engagement portion 126. In some embodiments, the rod head 122 is formed as a disc-shaped flange disposed at an upper end of the plunger rod 108. The rod head 122 is configured to provide an interface to the user to push on to advance the plunger rod 108 farther into the central bore of the barrel 104.

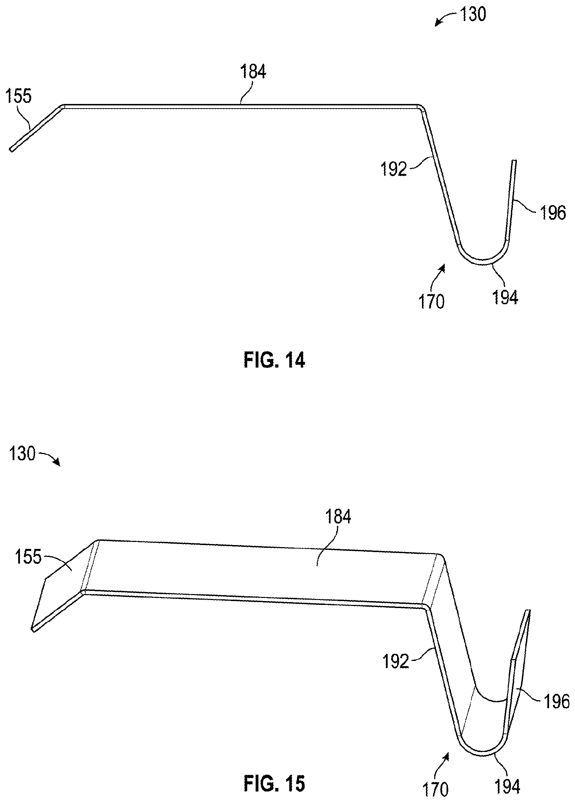

The ribbed portion 124 includes a plurality of ribs 128 are configured to engage a user feedback mechanism (e.g., engagement member 130, shown in FIGS. 14-19). As will be described further below, as the plunger rod 108 is advanced, plurality of ribs 128 are configured to contact the engagement member 130, which provides both tactile and audible feedback to the user to control a quantity of the substance dispensed from the syringe 100 during use.

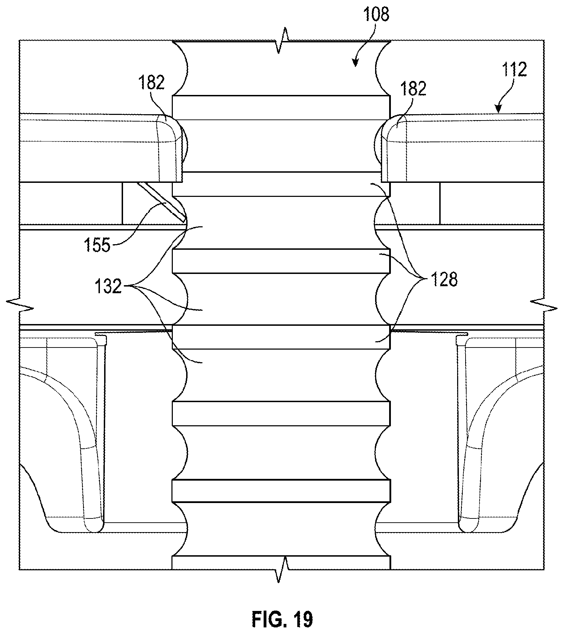

As best illustrated in FIG. 19, the plurality of ribs 128 are separated by a plurality of recesses 132. In some embodiments, as shown in FIG. 19, each of the plurality of ribs 128 may comprise a generally flat outer profile. However, in some other embodiments, each of the plurality of ribs 128 may alternatively form a generally rounded (e.g., convex) outer profile or may form a single pointed edge between adjacent recesses 132, as desired for a given application. In some embodiments, as shown in FIG. 19, each of the plurality of recesses 132 may comprise a generally rounded (e.g., concave) outer profile. In some other embodiments, each of the plurality of recesses 132 may alternatively form a rectangular outer profile, a semi-circular outer profile, a triangular outer profile, or any other suitably-shaped outer profile, as desired for a given application.

Referring again to FIG. 9, the plunger engagement portion 126 is configured to removably receive the plunger or seal 120. Specifically, when assembled, the plunger or seal 120 is configured to be slid over the plunger engagement portion 126. That is, the plunger or seal 120 may be formed of a flexible material and have an opening formed therein, such that the plunger or seal 120 can be pushed onto the plunger engagement portion 126 prior to use and subsequently pulled off of the plunger engagement portion 126 to allow for the plunger or seal 120 to be replaced.

In some instances, the plunger rod 108 may be formed of a hard plastic material to aid in maintaining the plunger rod 108 in axial alignment with the barrel 104 during use. For example, the plunger rod 108 may be formed of acrylonitrile butadiene styrene, polyamide, polypropylene, polycarbonate, or any other suitable hard plastic material, as desired for a given application. In some instances, the plunger rod 108 may further be formed using an injection molding process. In some other instances, the plunger rod 108 may be formed using any other suitable manufacturing technique.

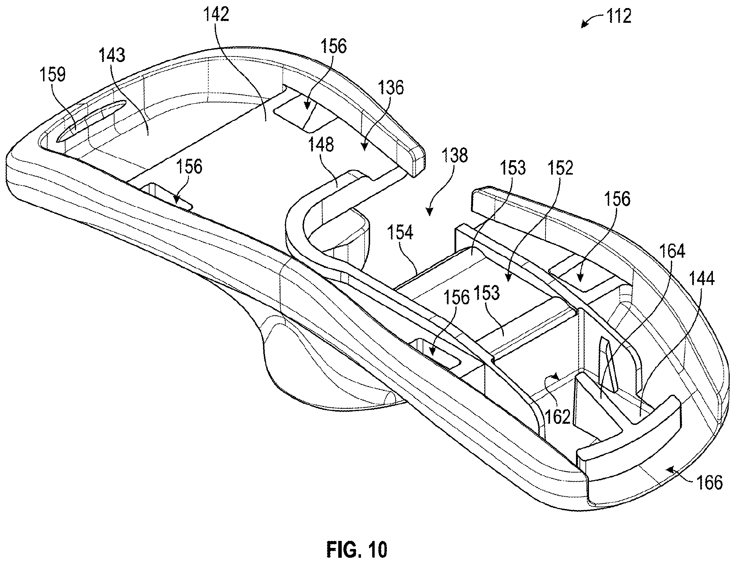

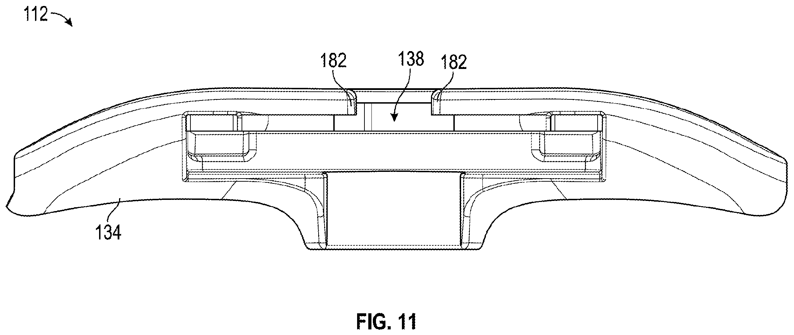

Referring now to FIGS 10 and 11, the lower grip component 112 includes a lower gripping surface 134, an upper receiving section 136, and a central opening 138. As best illustrated in FIG. 10, the lower gripping surface 134 forms a pair of opposed gripping portions extending away from the central opening 138. Each gripping portion defines a shallow concavity formed as a generally saddle-shaped surface. Specifically, each gripping portion is generally concave along a longitudinal axis 140 (shown in FIG. 8) of the lower grip component 112 and generally convex in a direction perpendicular to the longitudinal axis 140. As such, the lower gripping surface 134 provides ergonomic gripping surfaces for the user to grasp from either lateral side while using the syringe 100.

The upper receiving section 136 is configured to retainably receive the upper grip component 114 when the syringe 100 is assembled. The upper receiving section 136 includes an upper recessed portion 142 disposed around the central opening 138, a first lower recessed portion 143 disposed adjacent to the upper recessed portion 142 at a first longitudinal end of the upper receiving section 136, and a second lower recessed portion 144 disposed adjacent to the upper recessed portion 142 at a second longitudinal end, opposite the first longitudinal end, of the upper receiving section 136.

The upper recessed portion 142 is configured to receive a coupling segment 146 of the upper grip component 114. The upper recessed portion 142 includes an interior support wall 148 extending around a portion of the perimeter of the central opening 138. When the syringe 100 is assembled, a lateral surface of the interior support wall 148 (i.e., a surface facing the central opening 138) is configured to contact a corresponding lateral surface of an interior support wall 150 of the upper grip component 114. Stated differently, the interior support wall 148 of the upper recessed portion 142 is configured to wrap around and partially envelop a portion of the interior support wall 150 of the upper grip component 114.

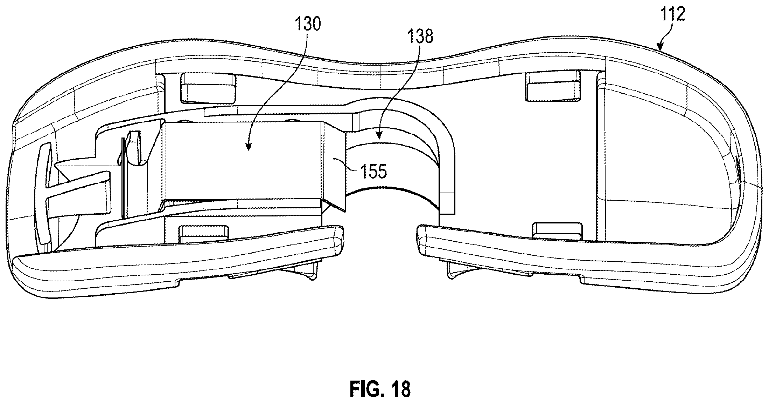

The upper recessed portion 142 further includes an engagement member slot 152 configured to receive the engagement member 130 (as shown in FIG. 18). The engagement member slot 152 includes a pair of lower support protrusions 153 configured to support the engagement member 130 when the engagement member 130 is received within the engagement member slot 152. The engagement member slot 152 further includes an angled end surface 154 disposed adjacent to the central opening 138 and configured to provide a clearance between the angled end surface 154 and a rod engagement segment 155 of the engagement member 130, as will be further discussed below.

With reference again to FIG. 10, the upper recessed portion 142 further includes a plurality of retention slots 156 configured to retainably receive a plurality of corresponding retention tabs 157 of the upper grip component 114 to retain the upper grip component 114 within the lower grip component 112 when the syringe 100 is assembled. In the illustrated embodiment shown in FIG. 10, the upper recessed portion 142 includes four retention slots 156 disposed about a perimeter of the upper recessed portion 142. However, in other embodiments, the upper recessed portion 142 may include more or fewer than four retention slots and/or the retention slots may be arranged differently, as desired for a given application.

As shown in FIG. 10, the first lower recessed portion 143 is recessed farther into the upper receiving section 136 than the upper recessed portion 142. The first lower recessed portion 143 is configured to receive a protruding portion 158 of the coupling segment 146 of the upper grip component 114. The first lower recessed portion 143 further includes a retention recess 159 within a first longitudinal wall at the first longitudinal end of the upper receiving section 136. The retention recess 159 is configured to engage a corresponding retention protrusion 160 of the upper grip component 114 when the syringe 100 is assembled to further aid in retaining the upper grip component 114 within the lower grip component 112.

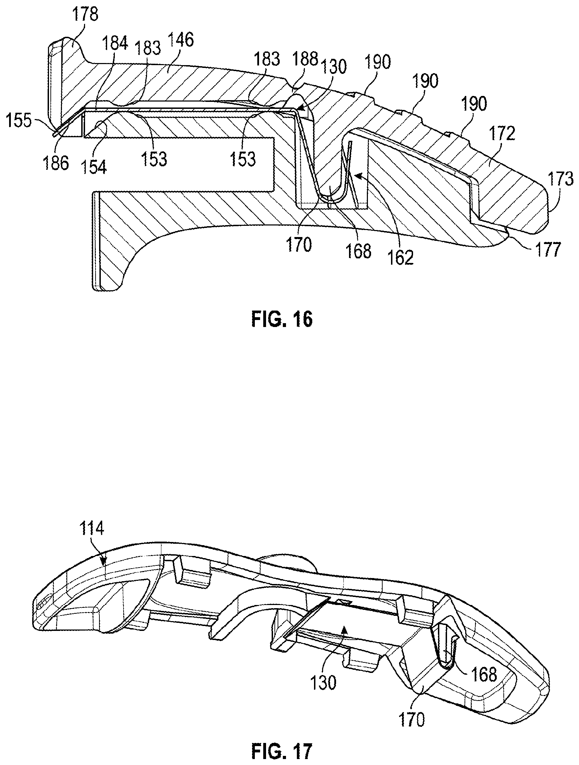

The second lower recessed portion 144 is similarly recessed farther into the upper receiving section 136 than the upper recessed portion 142. The second lower recessed portion 144 includes an activation member cavity 162, an activation button engagement protrusion 164, and an activation button clearance opening 166. As best shown in FIG 16, the activation member cavity 162 is configured to receive and provide a clearance for an activation member 168 of the upper grip component 114 and an activation segment 170 of the engagement member 130 when the syringe 100 is assembled.

The activation button engagement protrusion 164 protrudes upward from a lower surface of the second lower recessed portion 144. In some embodiments, the activation button engagement protrusion 164 is configured to come into contact with (e.g. configured to abut against) an activation button segment 172 of the upper grip component 114 when the syringe 100 is assembled and the activation button segment 172 is pressed down on by the user, as will be discussed further below.

The activation button clearance opening 166 extends through the second longitudinal end of the upper receiving section 136. Accordingly, the activation button clearance opening 166 provides clearance for a tip 173 (shown in FIG. 16) of the activation button segment 172 to extend longitudinally outward past a longitudinal end 177 of the lower grip component 112, such that the user may pull upward on the activation button segment 172, as will be discussed further below.

The central opening 138 of the lower grip component 112 extends vertically throughout the entire lower grip component 112 (e.g., from the upper receiving section 136 through the lower gripping surface 134) and laterally into a lateral side of the lower grip component 112. The central opening 138 is further configured to receive the plunger rod 108 and the barrel 104 when the syringe 100 is assembled. Specifically, as best illustrated in FIG. 11, the central opening 138 includes a lower portion configured to laterally receive the barrel 104, a central portion configured to laterally receive the barrel flange 118, and an upper portion configured to laterally receive the plunger rod 108. In some instances, the central opening 138 may be sized to provide a slight interference fit with the barrel 104, the barrel flange 118, and/or the plunger rod 108 to help retain the barrel 104, the barrel flange 118, and/or the plunger rod 108 within the central opening 138 when the syringe 100 is assembled.

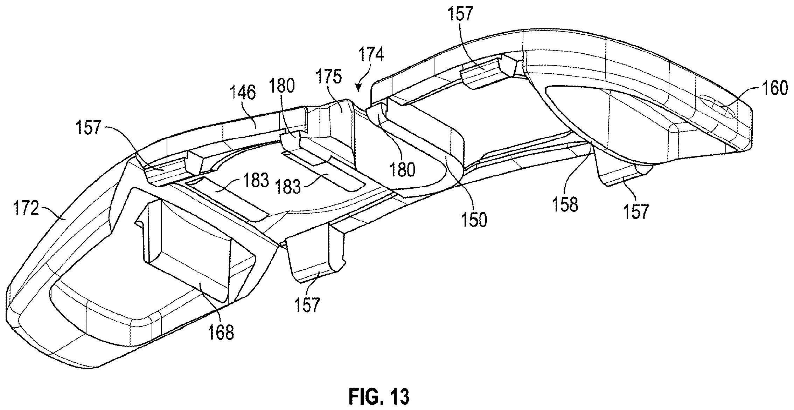

The upper grip component 114 is configured to be retainably received within the upper receiving section 136 of the lower grip component 112. As best illustrated in FIG. 13, the upper grip component 114 includes the coupling segment 146 and the activation button segment 172. The coupling segment 146 includes a central opening 174 configured to align with the central opening 138 of the lower grip component 112 to form the central grip opening 110 (shown in FIG. 1).

In some instances, the central opening 174 includes a pair of opposed protrusions 175 near a lateral end of the central opening 174. The pair of opposed protrusions 175 are configured to collectively form a gap that is slightly smaller than a diameter of the plunger rod 108. When the syringe 100 is assembled, the plunger rod 108 may be pressed laterally into the central opening 174, through the pair of opposed protrusions 175 and into a rod receiving portion 176. Accordingly, the pair of opposed protrusions 175 may provide a snap-fit retention of the plunger rod 108 within the rod receiving portion 176. The rod receiving portion 176 may define a semi-circular shape having a diameter sized to allow for the plunger rod 108 to be moved up or down within the central opening 174.

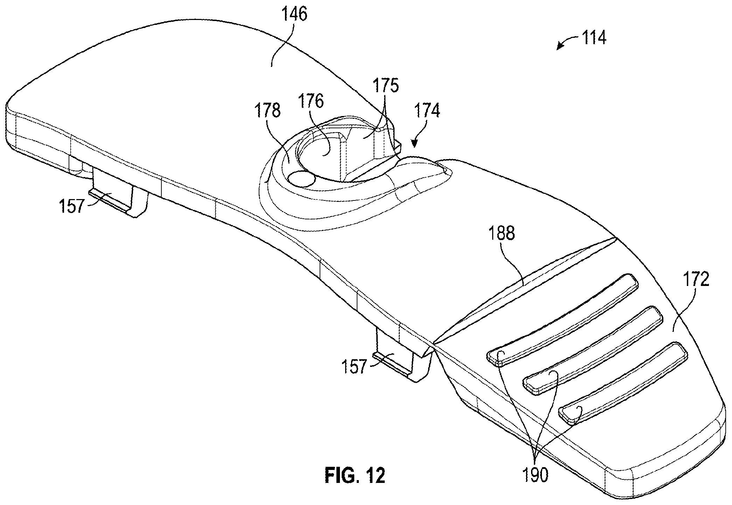

As best shown in FIG. 12, the central opening 174 of the upper grip component 114 is partially surrounded by a support lip 178. The support lip 178 protrudes vertically away from an upper surface of the coupling segment 146 adjacent the central opening 174. The support lip 178 is configured to support the plunger rod 108 such that it is maintained in axial alignment with the central opening 174 and the barrel 104 during use. It should be appreciated that, in some embodiments, the support lip 178 may protrude farther in the vertical direction than illustrated in FIG. 12 to provide additional support to the plunger rod 108 during use. For example, in some instances, the support lip 178 may protrude between 1 mm and 20 mm vertically from the upper surface of the coupling segment 146.

Additionally, as best shown in FIG. 13, the interior support wall 150 of the coupling segment 146 extends downward from a lower surface of the coupling segment 146 adjacent the central opening 174. Accordingly, the interior support wall 150 is similarly configured to provide additional support to the plunger rod 108 to maintain axial alignment with the barrel 104 during use.

When the upper grip component 114 is inserted into the upper receiving section 136 of the lower grip component 112, the retention protrusion 160 and the retention tabs 157 of the upper grip component 114 are configured to engage the retention recess 159 and the retention slots 156, respectively, of the lower grip component 112 to retain the upper grip component within the lower grip component 112. Further, the upper grip component 114 further includes a pair of lateral protrusions 180 (shown in FIG. 13) extending away from a lateral side of the upper grip component 114, on opposite sides of the central opening 174. When assembled, the pair of lateral protrusions 180 of the upper grip component 114 are further configured to be inserted below corresponding sidewall ends 182 (shown in FIG. 11) disposed on opposing sides of the central opening 138 of the lower grip component 112, thereby further retaining the upper grip component 114 within the lower grip component 112.

As best shown in FIG. 16, the coupling segment 146 further includes a pair of upper protrusions 183 configured to align with the pair of lower support protrusions 153 of the engagement member slot 152. When the syringe 100 is assembled, a top segment 184 of the engagement member 130 is configured to be arranged between the pair of lower support protrusions 153 of the engagement member slot 152 and the pair of upper protrusions 183 of the coupling segment 146.

Further, a gap formed between the upper protrusions 183 and the lower support protrusions 153 is configured to allow for the top segment 184 (and thus the entire engagement member 130) to slide toward or away from the central grip opening 110. Additionally, because the top segment 184 of the engagement member 130 only contacts the lower support protrusions 153 and, in some instances, the upper protrusions 183 (as opposed to an elongated lower and/or upper flat surface), the friction between the engagement member 130 and the lower and upper grip components 112, 114 is reduced, thereby improving the ease of activation of the syringe grip assembly 102 (e.g., moving the engagement member 130 between an engaged position and a disengaged position) during use.

Referring still to FIG. 16, the coupling segment 146 further includes an angled surface 186 configured to be generally parallel with and spaced apart from the angled end surface 154 of the engagement member slot 152. As depicted, a gap formed between the angled surface 186 and the angled end surface 154 is configured to provide sufficient clearance to allow for the rod engagement segment 155 (and thus the entire engagement member 130) to slide toward or away from the central grip opening 110 to allow for the rod engagement segment 155 to engage or disengage the plunger rod 108, as will be further discussed below.

With reference again to FIG. 12, the activation button segment 172 is connected to the coupling segment 146 by a joint 188. The joint 188 is configured to allow the activation button segment 172 to rotate with respect to the coupling segment 146 about the joint 188. As best shown in FIG. 16, the joint 188 is a thin, flexible segment that is formed continuously with the coupling segment 146 and the activation button segment 172. In this embodiment, the joint 188 is configured to flex or bend to allow for the activation button segment 172 to rotate with respect to the coupling segment 146. However, in some other embodiments, the joint 188 may alternatively be a hinge joint or any other suitable joint type, as desired for a given application.

The activation button segment 172 further includes the activation member 168 and a plurality of grip protrusions 190. As best illustrated in FIG. 16, when the syringe 100 is assembled, the activation member 168 is disposed within the activation member cavity 162 of the lower grip component 112. As shown in FIG. 17, the activation member 168 is further configured to engage the activation segment 170 of the engagement member 130 to selectively move the engagement member 130 between the engaged and the disengaged positions, as will be described further below.

The plurality of grip protrusions 190 protrude from an upper surface of the activation button segment 172 and are configured to aid the user in activating the syringe grip assembly 102. In the illustrated embodiment, the activation button segment 172 includes three grip protrusions 190. However, it should be appreciated that the activation button segment 172 may include more or less grip protrusions 190, as desired for a given application. For example, in some instances, the activation button segment 172 may include between one and five activation grip protrusions 190.

In some instances, the lower grip component 112 and/or the upper grip component 114 may be formed of a plastic material, such as acrylonitrile butadiene styrene, polyolefin, polyamide, polyethylene, polypropylene, polycarbonate, or any other suitable plastic material, as desired for a given application. In some instances, the lower grip component 112 and/or the upper grip component 114 may further be formed using an injection molding process. In some other instances, the lower grip component 112 and/or the upper grip component 114 may be formed using any other suitable manufacturing technique.

Referring now to FIGS. 14 and 15, the engagement member 130 includes the rod engagement segment 155, the activation segment 170, and the top segment 184. The rod engagement segment 155 is disposed at a first longitudinal end of the top segment 184 and is angled downward with respect to the top segment 184. The top segment 184 extends between the rod engagement segment 155 and the activation segment 170. The activation segment 170 is disposed at a second longitudinal end of the top segment 184, opposite the rod engagement segment 155. The activation segment 170 includes a first wall 192, a curved bottom wall 194, and a second wall 196. The first wall 192 extends at a downward angle away from the top segment 184 and is coupled to the curved bottom wall 194. The curved bottom wall 194 curves in an upward direction and is further coupled to the second wall 196. The second wall 196 is coupled to and extends generally upward from the curved bottom wall 194. Accordingly, the activation segment 170 defines a generally "U"-shaped or "V"-shaped profile.

In some instances, the engagement member 130 may be formed of a metallic material. For example, the engagement member 130 may be formed using a sheet metal stamping process. In some other instances, the engagement member 130 may alternatively be formed of a plastic material or any other suitable material, as desired for a given application.

Now that the various components of the syringe 100 and the syringe grip assembly 102 have been described above, a general method of assembling and using the syringe 100 and the syringe grip assembly 102 will be described below. It should be appreciated that the following method is provided as an example and is not meant to be limiting.

During assembly, the upper grip component 114 may first be inserted or "snapped" into the upper receiving section 136 of the lower grip component 112, with the engagement member 130 disposed within the engagement member slot 152 between the lower and upper grip components 112, 114, as described above, to assemble the syringe grip assembly 102.

With the syringe grip assembly 102 assembled, the barrel 104 (e.g., including the barrel flange 118) is then inserted laterally into the central grip opening 110. In some embodiments, the plunger rod 108 may be inserted into the barrel 104 prior to insertion of the barrel 104 into the central grip opening 110, such that the barrel 104 and the plunger rod 108 are inserted laterally into the central grip opening 110 at the same time. In some other embodiments, the plunger rod 108 may be inserted into the barrel 104 after the barrel 104 has already been inserted into the central grip opening 110 (e.g., vertically through the rod receiving portion 176 of the central opening 174 of the upper grip component 114).

With the barrel 104 and the plunger rod 108 inserted into the syringe grip assembly 102, the syringe 100 may then be used to dispense a substance from within the barrel 104 and out through the needle 106. In some instances, the syringe 100 may be provided with the plunger rod 108 already inserted into the barrel 104 and a substance already drawn into the barrel 104 (e.g., pre-filled). In some other instances, the barrel 104 may be provided empty, and a substance can be drawn in after insertion of the barrel 104 and the plunger rod 108 into the syringe grip assembly 102.

In any case, the syringe grip assembly 102 is selectively actuatable between an activated or engaged position, in which the rod engagement segment 155 of the engagement member 130 is pushed into contact with the plunger rod 108, and a deactivated or disengaged position, in which the rod engagement segment 155 of the engagement member 130 is pulled out of contact with the plunger rod 108.

Specifically, to actuate the syringe grip assembly 102 into the activated or engaged position, the user may press downward on the upper surface of the activation button segment 172 to move the activation button segment 172 into an activated or lowered (first) position. As best depicted in FIG. 16, as the activation button segment 172 is pressed downward, the activation member 168 rotates into the first wall 192 of the activation segment 170, thereby forcing the entire engagement member 130 toward the central grip opening 110. As the engagement member 130 is forced toward the central grip opening 110, the rod engagement segment 155 eventually comes into contact with the plunger rod 108, as shown in FIG. 19.

In some embodiments, if the gap between lower support protrusions 153 and the upper protrusions 183 is sufficiently large, as the activation member 168 rotates into the first wall 192, the top segment 184 may tilt about the lower support protrusion 153 closest to the activation button segment 172, such that the rod engagement segment 155 is also tilted upward into contact with the plunger rod 108.

With the syringe grip assembly 102 in the activated or engaged position, as the user dispenses substance from the syringe 100, the syringe grip assembly 102 is configured to provide tactile and/or auditory feedback to the user indicative of the volume of substance that is being dispensed. Specifically, with the rod engagement segment 155 in contact with the plunger rod 108, as the plunger rod 108 is advanced into the barrel 104 to dispense the substance, the rod engagement segment 155 sequentially "snaps" or "clicks" between subsequent recesses 132 (e.g., over each subsequent rib 128). As the rod engagement segment 155 "snaps" or "clicks" between subsequent recesses 132, the user senses (e.g., by feeling and/or hearing) each "snap" or "click," thereby informing the user as to how far the plunger rod 108 has been advanced into the barrel 104.

Specifically, based on the sizing of the ribs 128 and the recesses 132, each "snap" or "click" corresponds to a predetermined amount of substance being dispensed. For example, in some instances, the ribs 128 and recesses 132 may be sized such that each "snap" or "click" corresponds to approximately 10 mL of substance being dispensed. In other instances, the ribs 128 and recesses 132 may be sized such that each "snap" or "click" corresponds to approximately 25 mL of substance being dispensed. In other instances, the ribs 128 and recesses 132 may be sized such that each "snap" or "click" corresponds to various other quantities of substances being dispensed, as desired for a given application.

As best illustrated in FIG. 19, in some instances, the rod engagement segment 155 being angled with respect to the central axis of the central grip opening 110 and the plunger rod 108 (e.g., provided at a non-normal angle, that is, an angle other than a right angle) provides a more smooth and audible "snap" or "click" between subsequent recesses 132 as opposed to a rod engagement segment 155 that is normal to the central axis of the plunger rod 108. In some instances, as discussed above, each of the plurality of ribs 128 may have a generally rounded (e.g., convex) outer profile or may form a single pointed edge between adjacent recesses 132. In these instances, as the rod engagement segment 155 is pushed into contact with the plunger rod 108, the rounded or pointed rib 128 naturally pushes the rod engagement segment 155 into an adjacent recess 132, as opposed to the rod engagement segment 155 possibly getting stuck on the outer surface of the rib 128.

Additionally, when the engagement member 130 is in the activated or engaged position, the rod engagement segment 155 is configured to prevent the plunger rod 108 from being retracted or pulled out of the barrel 104. That is, when the plunger rod 108 is pulled out of the barrel 104, the rod engagement segment 155 is substantially perpendicular to a lower surface of the curved recess 132. Accordingly, the rod engagement segment 155 effectively blocks the plunger rod 108 from moving upward when the engagement member 130 is in the activated or engaged position. This may effectively prevent accidental aspiration during an injection process by preventing the user from accidentally pulling the plunger rod 108 back during use.

After the user has finished using the syringe 100 to dispense the substance, the user may then actuate the syringe grip assembly 102 into the deactivated or disengaged position by pulling up on the tip 173 of the activation button segment 172 to move the activation button segment 172 into a deactivated or raised (second) position that is elevated with respect to the lowered (first) position. As best depicted in FIG. 16, as the user pulls upward on the tip 173, the activation member 168 of the activation button segment 172 is rotated into the second wall 196 of the activation segment 170, thereby pulling the entire engagement member 130 away from the central grip opening 110. As the engagement member 130 is pulled away from the central grip opening 110, the rod engagement segment 155 is comes out of contact with and is separated from the plunger rod 108, and the plunger rod 108 can move freely into and out of the barrel 104.

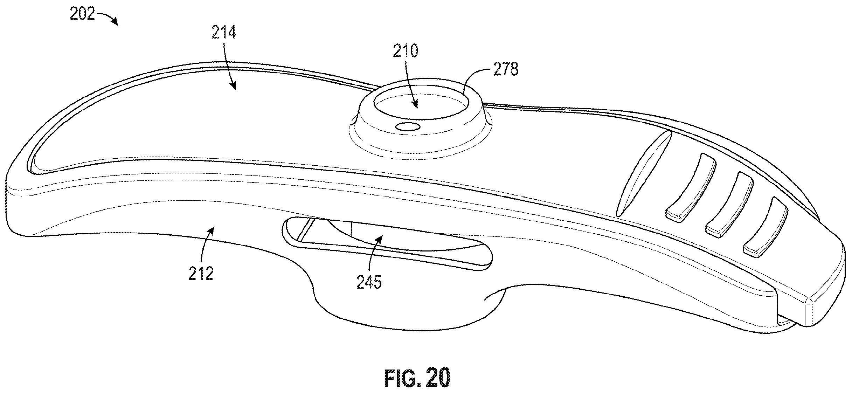

Referring now to FIGS. 20-25 generally, another syringe grip assembly 202 is shown, according to an example embodiment. The syringe grip assembly 202 is substantially similar to the syringe grip assembly 102 in structure and function. As such, it will be appreciated that the description above, with respect to the syringe grip assembly 102, can be similarly applied to the syringe grip assembly 202. Accordingly, the following description will focus on the differences between the syringe grip assembly 202 and the syringe grip assembly 102.

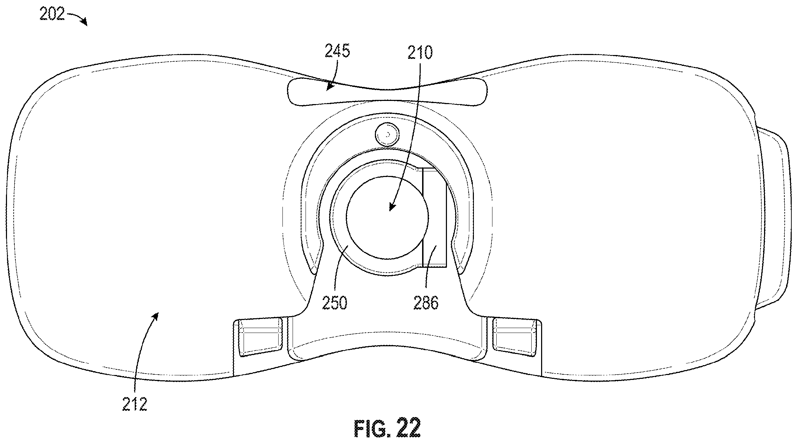

For example, the syringe grip assembly 202 is similarly configured to receive a plunger rod (e.g., the plunger rod 108 discussed above) within a central grip opening 210 formed by a lower grip component 212 and an upper grip component 214 of the syringe grip assembly 202. However, different from central grip opening 110 of the syringe grip assembly 102, the central grip opening 210 of the syringe grip assembly 202 does not extend through a lateral side of the syringe grip assembly 202. Instead, as shown in FIG. 22, the central grip opening 210 forms a generally circular bore extending vertically throughout the syringe grip assembly 202.

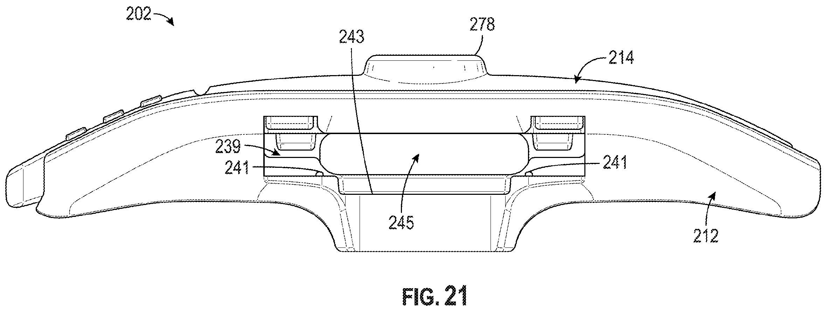

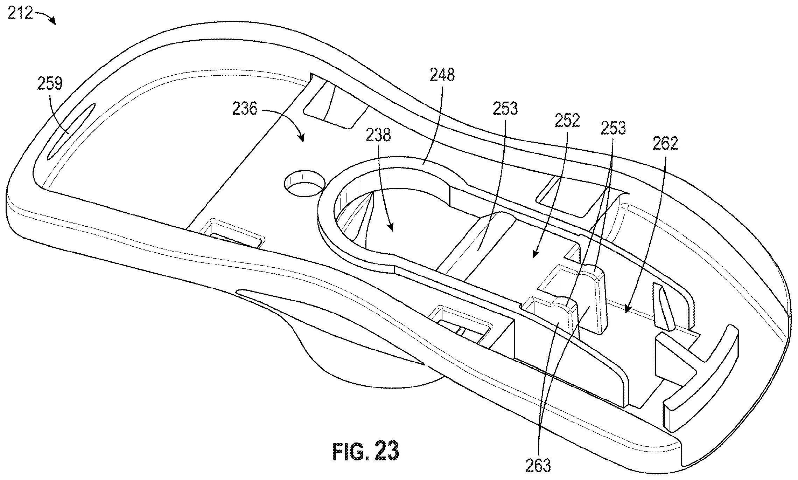

With reference to FIG. 23, the lower grip component 212 includes a central opening 238 extending vertically throughout the entire lower grip component 212. However, unlike the central opening 138 of the lower grip component 112, the central opening 238 does not extend laterally out of a lateral side of the lower grip component 212. Instead, as best shown in FIG. 21, to allow for the insertion of a barrel (e.g., the barrel 104), the lower grip component 212 includes a lateral barrel slot 239 configured to receive a barrel (e.g., the barrel 104) when the syringe grip assembly 202 is assembled together with a syringe (e.g., the syringe 100). Specifically, the lateral barrel slot 239 includes a lower barrel portion configured to laterally receive a barrel (e.g., the barrel 104) and an upper barrel flange portion configured to receive a barrel flange (e.g., the barrel flange 118).

In some instances, as shown in FIG. 21, the lateral barrel slot 239 further includes interference ribs 241 arranged on a lower surface of the upper barrel flange portion of the lateral barrel slot 239. In some instances, the interference ribs 241 are configured to provide a slight interference fit between the lateral barrel slot 239 and the barrel flange when the barrel flange and barrel are inserted into the lateral barrel slot 239 to aid in retaining the barrel and barrel flange in engagement with the syringe grip assembly 202.

With continued reference to FIG. 21, the lower grip component 212 further includes a barrel-end lip 243 arranged at an edge formed between the upper barrel flange portion and the lower barrel portion of the lateral barrel slot 239. In some instances, the barrel-end lip 243 provides clearance for a corresponding ridge feature of certain syringes arranged between the barrel and the barrel flange. However, in some instances, a syringe utilized with the syringe grip assembly 202 may not include this ridge. Accordingly, in these instances, the lower grip component 212 may not include the barrel-end lip 243.

Further, in some embodiments, the lower grip component 212 includes a lateral opening 245 extending laterally throughout the lower grip component 212 from an inner surface of the lateral barrel slot 239 through an opposite lateral side of the lower grip component 212. In some instances, the lateral opening 245 may be included to reduce an amount of material utilized when forming the lower grip component 212. Accordingly, in some instances, the lateral opening 245 may be omitted from the lower grip component 212, similar to the lower grip component 112 of the syringe grip assembly 102.

The syringe grip assembly 202 similarly includes an engagement member (e.g., the engagement member 130) disposed between the lower grip component 212 and the upper grip component 214 to provide the same "snap" or "click" functionality discussed above, with respect to the syringe grip assembly 102. Accordingly, as shown in FIG. 23, an upper receiving section 236 of the lower grip component 212 similarly includes an engagement member slot 252 extending from the central opening 238 to an activation member cavity 262. The engagement member slot 252 similarly includes lower support protrusions 253. However, the lower support protrusion 253 nearest the activation member cavity 262 is formed by a pair of flanges 263 that extend partially into the activation member cavity 262. Additionally, as the central opening 238 does not extend laterally out of a lateral side of the lower grip component 212, an interior support wall 248 extends around a perimeter of the central opening 238 between opposite lateral sides of the engagement member slot 252.

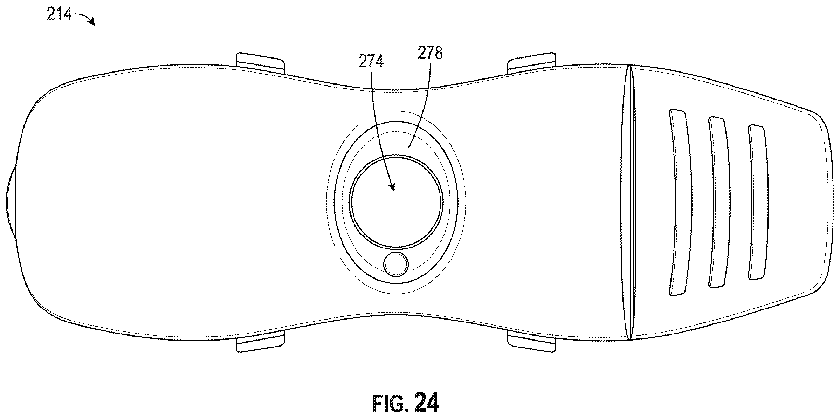

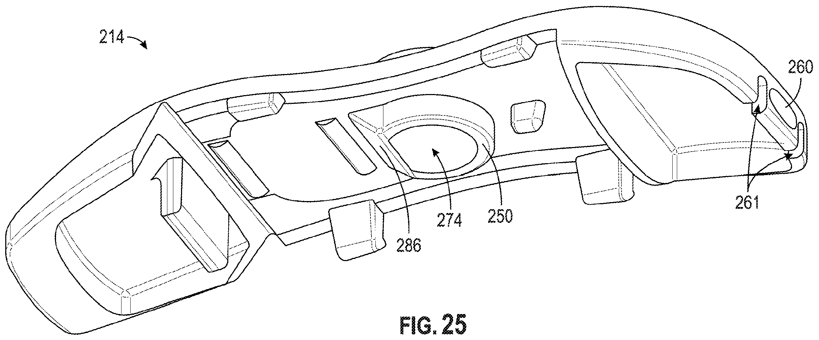

Referring now to FIGS. 24 and 25, the upper grip component 214 is similarly configured to be retainably received within the upper receiving section 236 of the lower grip component 212. The upper grip component 214 similarly includes a central opening 274 extending vertically through the upper grip component 214 and configured to align with the central opening 238 of the lower grip component 212 to form the central grip opening 210 (as shown in FIG. 20). However, unlike the central opening 174 of the upper grip component 114, the central opening 274 of the upper grip component 214 does not extend laterally out of a lateral side of the upper grip component 214. Instead, as best shown in FIG. 24, the central opening 274 forms a generally circular through-hole extending vertically through the upper grip component 214. Accordingly, a support lip 278 of the upper grip component 214 completely surrounds the central opening 274. By completely surrounding the central opening 274, the support lip 278 may provide additional support, as compared to the support lip 178, to maintain the axial alignment of a plunger rod (e.g., the plunger rod 108) with the central opening 274 during use.

As shown in FIG. 25, an interior support wall 250 extends around a perimeter of the central opening 274 between lateral sides of an angled surface 286. Similar to the angled surface 186 of the upper grip component 114, the angled surface 286 of the upper grip component 214 is configured to provided clearance for the engagement member (e.g., the engagement member 130) when the syringe grip assembly 202 is assembled. Further, as best shown in FIGS. 22, 23, and 25, the interior support wall 250 and angled surface 286 of the upper grip component 214 are configured to be received within the central opening 238 of the lower grip component 212 (shown in FIG. 23), with the interior support wall 250 of the upper grip component 214 arranged adjacent to the interior support wall 248 of the lower grip component 212.

Additionally, as shown in FIG. 25, the upper grip component 214 similarly includes a retention protrusion 260 configured to engage a corresponding retention recess 259 of the lower grip component 212 (shown in FIG. 23). However, the upper grip component 214 further includes a pair of notches 261 arranged on either side of the retention protrusion 260 to allow for the retention protrusion 260 (e.g., the portion of the upper grip component 214 between the pair of notches 261) to more easily flex inwardly (e.g., toward the central opening 274) when the upper grip component 214 is inserted into the upper receiving section 236 of the lower grip component 212 during assembly.

Accordingly, it will be appreciated that the syringe grip assembly 202 may be used in a similar manner to the syringe grip assembly 102 discussed above. In some instances, the syringe grip assembly 102 and the syringe grip assembly 202 may be used interchangeably with the same syringe, as desired for a given application. For example, the syringe 100 and the syringe grip assembly 102 or the syringe grip assembly 202 disclosed herein may be utilized to inject known amounts of substances into patients or subjects for a variety of medical procedures or treatments. For example, in some instances, the syringe 100 and the syringe grip assembly 102 or the syringe grip assembly 202 may be configured to provide injections of insulin. In other instances, the syringe 100 and the syringe grip assembly 102 or the syringe grip assembly 202 may be utilized to provide precise injections of a variety of other substances.

For example, in some instances, the syringe 100 and the syringe grip assembly 102 or the syringe grip assembly 202 may be utilized to deliver injections of various dermal fillers for cosmetic use, such as, for example, cross-linked or non-cross-linked hyaluronic acid gels, collagen, calcium hydroxyl apatite, poly-L-lactic acid (PLLA), other polysaccharides and polymethylmethacrylate (PMMA). The syringe 100 and the syringe grip assembly 102 or the syringe grip assembly 202 may further be utilized to deliver precise injections of various liquid compositions comprising active substances and/or bioactive agents, such as local anesthetics, cicatrizants, antioxidants, botulinum toxin, hormones, and/or combinations of the foregoing. For example, one liquid composition of this type may be a gel composition with a hyaluronic acid gel carrier and an active substance and/or a bioactive agent, e.g., a local anesthetic or a cicatrizant, such as dextranomer beads, and/or any combination of the foregoing.

It should be appreciated that, in some embodiments, the syringe grip assembly 102 or the syringe grip assembly 202 and the plunger rod 108 may be provided separately from the barrel 104. For example, in some instances, the syringe grip assembly 102 or the syringe grip assembly 202 and the plunger rod 108 may be designed, sized, and configured to retainably receive and engage a standard-sized barrel for a desired substance (e.g., a standard insulin syringe barrel). Accordingly, in some instances, the central grip opening 110 or the central grip opening 210 may be larger or smaller, or may be shaped differently to receive a different type of barrel depending on the intended application. Similarly, in some instances, the plunger rod 108 may define a larger or smaller diameter to correspond to the size of the central bore of the type of barrel being utilized.

It should further be appreciated that, in some instances, various components of the syringe grip assembly 102, the syringe grip assembly 202, and/or the plunger rod 108 may be formed differently than depicted in the figures. For example, in some instances, if the plunger rod 108 is to be injection molded and needs to be wider to match a given barrel size, the plunger rod 108 may be formed as a hollow component (e.g., having a hollow axial core) to avoid either increasing cooling time or risking warping after molding. In these instances, the hollow axial core may then be capped at the rod head 122.

Additionally, in some embodiments, the syringe grip assembly 102 may be formed with the central grip opening 110 extending only vertically throughout the center of the syringe grip assembly 102 (i.e., not extending laterally out the syringe grip assembly 102). In these instances, each of the lower and upper grip components 112, 114 may be provided in two halves (e.g., longitudinal or latitudinal) that are configured to be selectively coupled utilizing retention slots and corresponding retention tabs (e.g., similar to the retention slots 156 and the retention tabs 157 described herein). Accordingly, when assembled, the two halves of each of the lower and upper grip components 112, 114 may be coupled together around the barrel 104, the plunger rod 108, and the engagement member 130 before inserting the upper grip component 114 into the lower grip component 112, as discussed above, thereby eliminating the need for a lateral opening.

Although the figures may show a specific order of method steps, the order of the steps may differ from what is depicted. In some embodiments, two or more steps may be performed concurrently or with partial concurrence. All such variations are within the scope of the disclosure. Additionally, while various aspects and embodiments have been disclosed herein, other aspects and embodiments will be apparent to those skilled in the art. The various aspects and embodiments disclosed herein are for purposes of illustration and are not intended to be limiting, with the scope of the invention being indicated by the following claims.

## Claims

1. A syringe grip assembly (100) configured to receive a syringe barrel (104), the syringe grip assembly comprising:
a plunger rod (108) having a ribbed outer surface;
a syringe grip (102) including an activation button segment (172) and a central opening (138) extending through the syringe grip and being configured to receive the plunger rod (108); and
an engagement member (130) including a rod engagement segment (155), the engagement member (130) being selectively moveable using the activation button segment (172) between (i) an engaged position, in which the rod engagement segment (155) is in contact with the plunger rod (108), and (ii) a disengaged position, in which the rod engagement segment (155) is separated from the plunger rod (108);
wherein the activation button segment (172) is moveable between a lowered position, in which the engagement member (130) is moved to the engaged position, and a raised position, in which the engagement member (130) is moved to the disengaged position, **characterised in that** the rod engagement segment (155) is arranged at a non-normal angle with respect to a central axis of the plunger rod (108).

2. The syringe grip assembly of claim 1, wherein the ribbed outer surface includes a plurality of ribs (128) and, when the engagement member (130) is in the engaged position, the engagement member (130) is configured to allow the plunger rod (108) to move through the central opening (138) in a first direction and, as the plunger rod (108) moves in the first direction, the rod engagement segment (155) is configured to sequentially snap over each rib of the plurality of ribs.

3. The syringe grip assembly of claim 2, wherein the syringe grip is configured to provide at least one of tactile feedback or audible feedback to a user each time the rod engagement segment (155) snaps over a rib of the plurality of ribs (128), the at least one of tactile feedback or audible feedback being indicative of an amount of substance being dispensed through the syringe barrel.

4. The syringe grip assembly of any of the preceding claims, wherein the syringe grip includes a support lip (178) adjacent to the central opening (138) and configured to provide support to the plunger rod (108) such that the plunger rod (108) is maintained in axial alignment with the central opening (138).

5. The syringe grip assembly of any of the preceding claims, wherein the activation button segment (172) includes at least one grip protrusion on an external surface of the activation button segment (172).

6. The syringe grip assembly of claim 1, wherein the syringe grip includes a lower grip component (112) and an upper grip component (114), the lower grip component (112) including a lower gripping surface (134) and a receiving section, the upper grip component (114) received within the receiving section and including the activation button segment (172).

7. The syringe grip assembly of claim 6, wherein the receiving section of the lower grip component (112) includes an opening extending through a longitudinal end of the receiving section, and the activation button segment (172) extends beyond the longitudinal end of the receiving section such that the activation button segment (172) is accessible from a top surface and a bottom surface to allow for the activation button segment (172) to be selectively moved between the raised position and the lowered position.

8. The syringe grip assembly of any of claims 1-3, wherein the syringe barrel (104) is a 0.5 ml glass syringe barrel.

9. The syringe grip assembly of any of claims 1-3, wherein the syringe grip includes a lower gripping surface (134) defining at least one concavity.

10. The syringe grip assembly of any of claims 1-3, wherein, when the engagement member (130) is in the engaged position, the engagement member (130) is configured to allow the plunger rod (108) to move through the central opening (138) in a first direction and to prevent the plunger rod (108) from moving through the central opening (138) in a second direction opposite to the first direction.

11. The syringe grip assembly of any of claims 1-3, wherein the plunger rod (108) is formed of plastic selected from the group consisting of acrylonitrile butadiene styrene, polyamide, polypropylene, and polycarbonate.

12. The syringe grip assembly of claim 6, wherein the lower gripping surface (134) comprises a pair of opposed gripping portions extending away from the central opening (138).

13. The syringe grip assembly of claim 12, wherein each of the pair of opposed gripping portions is generally concave along a longitudinal axis of the lower grip component (112) and generally convex in a direction perpendicular to the longitudinal axis (140).

14. The syringe grip assembly of claim 1, wherein the plurality of ribs (128) are separated by a plurality of recesses (132).

## Patentansprüche

1. Spritzengriffbaugruppe (100), die zur Aufnahme eines Spritzenzylinders (104) ausgelegt ist, wobei die Spritzengriffbaugruppe umfasst:
eine Kolbenstange (108) mit einer gerippten Außenfläche;
einen Spritzengriff (102) mit einem Betätigungsknopfabschnitt (172) und einer zentralen Öffnung (138), die sich durch den Spritzengriff erstreckt und zur Aufnahme der Kolbenstange (108) ausgelegt ist; sowie
ein Eingriffselement (130) mit einem Stangeneingriffsabschnitt (155),
wobei das Eingriffselement (130) mithilfe des Betätigungsknopfabschnitts (172) wahlweise zwischen
(i) einer Eingriffsposition, in der das Stangen-Eingriffssegment (155) mit der Kolbenstange (108) in Kontakt steht, und
(ii) einer Ausrückposition, in der das Stangen-Eingriffssegment (155) von der Kolbenstange (108) getrennt ist;
wobei das Betätigungsknopfsegment (172) zwischen einer abgesenkten Position, in der das Eingriffselement (130) in die Eingriffsposition bewegt wird, und einer angehobenen Position, in der das Eingriffselement (130) in die Ausrückposition rückt, bewegbar ist,
**dadurch gekennzeichnet, dass** das Stangeneingriffssegment (155) in einem nicht senkrechten Winkel zur Mittelachse der Kolbenstange (108) angeordnet ist.

2. Spritzengriffbaugruppe nach Anspruch 1, wobei die gerippte Außenfläche eine Vielzahl von Rippen (128) umfasst und das Eingriffselement (130), wenn es sich in der Eingriffsposition befindet, so ausgebildet ist, dass es der Kolbenstange (108) ermöglicht, sich durch die zentrale Öffnung (138) in einer ersten Richtung zu bewegen, und während sich die Kolbenstange (108) in der ersten Richtung bewegt, ist das Stangen-Eingriffssegment (155) so ausgebildet, dass es nacheinander über jede der mehreren Rippen einrastet.

3. Spritzengriffanordnung nach Anspruch 2, wobei der Spritzengriff so ausgebildet ist, dass er einem Benutzer jedes Mal, wenn das Stangen-Eingriffssegment (155) über eine der mehreren Rippen (128) einrastet, mindestens entweder taktiles oder akustisches Feedback liefert, wobei das mindestens eine der beiden Feedbackarten - taktisches oder akustisches - die Menge der durch den Spritzenzylinder abgegebenen Substanz anzeigt.

4. Spritzengriffanordnung nach einem der vorstehenden Ansprüche, wobei der Spritzengriff eine Stützlippe (178) benachbart zur zentralen Öffnung (138) umfasst, die so ausgebildet ist, dass sie der Kolbenstange (108) Halt bietet, sodass die Kolbenstange (108) in axialer Ausrichtung mit der zentralen Öffnung (138) gehalten wird.

5. Spritzengriffbaugruppe nach einem der vorstehenden Ansprüche, wobei das Betätigungsknopfsegment (172) mindestens einen Griffvorsprung an einer Außenfläche des Betätigungsknopfsegments (172) aufweist.

6. Spritzengriffanordnung nach Anspruch 1, wobei der Spritzengriff ein unteres Griffteil (112) und ein oberes Griffteil (114) umfasst, wobei das untere Griffteil (112) eine untere Greiffläche (134) und einen Aufnahmebereich aufweist, wobei das obere Griffteil (114) in dem Aufnahmebereich aufgenommen ist und das Betätigungsknopfsegment (172) umfasst.

7. Spritzengriffanordnung nach Anspruch 6, wobei der Aufnahmebereich des unteren Griffelements (112) eine Öffnung aufweist, die sich durch ein Längsende des Aufnahmebereichs erstreckt, und sich das Betätigungsknopfsegment (172) über das Längsende des Aufnahmebereichs hinaus erstreckt, so dass das Betätigungsknopfsegment (172) von einer Oberseite und einer Unterseite aus zugänglich ist, um eine selektive Bewegung des Betätigungsknopfsegments (172) zwischen der angehobenen Position und der abgesenkten Position zu ermöglichen.

8. Spritzengriffanordnung nach einem der Ansprüche 1-3, wobei der Spritzenzylinder (104) ein 0,5-ml-Glas-Spritzenzylinder ist.

9. Spritzengriffanordnung nach einem der Ansprüche 1-3, wobei der Spritzengriff eine untere Griffoberfläche (134) aufweist, die mindestens eine Vertiefung definiert.

10. Spritzengriffanordnung nach einem der Ansprüche 1 bis 3, wobei das Eingriffselement (130), wenn es sich in der Eingriffsposition befindet, so ausgebildet ist, dass es eine Bewegung der Kolbenstange (108) durch die zentrale Öffnung (138) in einer ersten Richtung zulässt und eine Bewegung der Kolbenstange (108) durch die zentrale Öffnung (138) in einer zweiten Richtung, die der ersten Richtung entgegengesetzt ist.

11. Spritzengriffanordnung nach einem der Ansprüche 1 bis 3, wobei die Kolbenstange (108) aus einem Kunststoff gebildet ist, der aus der Gruppe ausgewählt ist, die aus AcrylnitrilButadien-Styrol, Polyamid, Polypropylen und Polycarbonat besteht.

12. Spritzengriffanordnung nach Anspruch 6, wobei die untere Greiffläche (134) ein Paar gegenüberliegender Greifabschnitte umfasst, die sich von der zentralen Öffnung (138) weg erstrecken.

13. Spritzengriffanordnung nach Anspruch 12, wobei jeder der beiden einander gegenüberliegenden Griffabschnitte entlang einer Längsachse des unteren Griffelements (112) im Wesentlichen konkav und in einer Richtung senkrecht zur Längsachse (140) im Wesentlichen konvex ist.

14. Spritzengriffanordnung nach Anspruch 1, wobei die mehreren Rippen (128) durch mehrere Aussparungen (132) voneinander getrennt sind.

## Revendications

1. Ensemble de poignée de seringue (100) conçu pour recevoir un corps de seringue (104), l'ensemble de poignée de seringue comprenant :
une tige de piston (108) présentant une surface extérieure nervurée ;
une poignée de seringue (102) comprenant un segment de bouton d'activation (172) et une ouverture centrale (138) qui s'étend à travers la poignée de seringue et est conçue pour recevoir la tige de piston (108) ; et
un élément d'engagement (130) comprenant un segment d'engagement de tige (155), l'élément d'engagement (130) pouvant être déplacé de manière sélective à l'aide du segment de bouton d'activation (172) entre (i) une position engagée, dans laquelle le segment d'engagement de tige (155) est en contact avec la tige de piston (108), et (ii) une position désengagée, dans laquelle le segment d'engagement de tige (155) est séparé de la tige de piston (108) ;
dans lequel le segment de bouton d'activation (172) est mobile entre une position abaissée, dans laquelle l'élément d'engagement (130) est déplacé vers la position engagée, et une position relevée, dans laquelle l'élément d'engagement (130) est déplacé vers la position désengagée,
**caractérisé en ce que** le segment d'engagement de tige (155) est disposé selon un angle non normal par rapport à un axe central de la tige de piston (108).

2. Ensemble de poignée de seringue selon la revendication 1,
dans lequel la surface extérieure nervurée comprend une pluralité de nervures (128) et, lorsque l'élément d'engagement (130) se trouve en position engagée, l'élément d'engagement (130) est conçu pour permettre à la tige de piston (108) de se déplacer à travers l'ouverture centrale (138) dans une première direction et, à mesure que la tige de piston (108) se déplace dans la première direction, le segment d'engagement de tige (155) est conçu pour s'enclencher successivement sur chacune des nervures de la pluralité de nervures.

3. Ensemble de poignée de seringue selon la revendication 2,
dans lequel la poignée de seringue est conçue pour fournir à un utilisateur au moins l'un parmi un retour tactile ou un retour sonore chaque fois que le segment d'engagement de tige (155) s'enclenche sur une nervure de la pluralité de nervures (128), ledit au moins un retour tactile ou retour sonore indiquant une quantité de substance distribuée à travers le corps de seringue.

4. Ensemble de poignée de seringue selon l'une des revendications précédentes,
dans lequel la poignée de seringue comprend une lèvre de support (178) adjacente à l'ouverture centrale (138) et conçue pour fournir un support à la tige de piston (108) de telle sorte que la tige de piston (108) soit maintenue en alignement axial avec l'ouverture centrale (138).

5. Ensemble de poignée de seringue selon l'une des revendications précédentes,
dans lequel le segment de bouton d'activation (172) comprend au moins une saillie de préhension sur une surface externe du segment de bouton d'activation (172).

6. Ensemble de poignée de seringue selon la revendication 1,
dans lequel la poignée de seringue comprend un composant de poignée inférieur (122) et un composant de poignée supérieur (114), le composant de poignée inférieur (112) présentant une surface de préhension inférieure (134) et une section de réception, le composant de poignée supérieur (114) étant logé dans la section de réception et comportant le segment de bouton d'activation (172).

7. Ensemble de poignée de seringue selon la revendication 6,
dans lequel la section de réception du composant de poignée inférieur (112) comprend une ouverture s'étendant à travers une extrémité longitudinale de la section de réception, et le segment de bouton d'activation (172) s'étend au-delà de l'extrémité longitudinale de la section de réception de telle sorte que le segment de bouton d'activation (172) soit accessible depuis une surface supérieure et une surface inférieure afin de permettre au segment de bouton d'activation (172) d'être déplacé de manière sélective entre la position relevée et la position abaissée.

8. Ensemble de poignée de seringue selon l'une des revendications 1 à 3,
dans lequel le corps de seringue (104) est un corps de seringue en verre de 0,5 ml.

9. Ensemble de poignée de seringue selon l'une des revendications 1 à 3,
dans lequel la poignée de seringue présente une surface de préhension inférieure (134) définissant au moins une concavité.

10. Ensemble de poignée de seringue selon l'une des revendications 1 à 3,
dans lequel, lorsque l'élément d'engagement (130) se trouve en position engagée, l'élément d'engagement (130) est conçu pour permettre à la tige de piston (108) de se déplacer à travers l'ouverture centrale (138) dans une première direction, et pour empêcher la tige de piston (108) de se déplacer à travers l'ouverture centrale (138) dans une deuxième direction opposée à la première direction.

11. Ensemble de poignée de seringue selon l'une des revendications 1 à 3,
dans lequel la tige de piston (108) est réalisée en une matière plastique choisie parmi le groupe constitué de l'acrylonitrile-butadiène-styrène, du polyamide, du polypropylène et du polycarbonate.

12. Ensemble de poignée de seringue selon la revendication 6,
dans lequel la surface de préhension inférieure (134) comprend une paire de parties de préhension opposées s'étendant en éloignement de l'ouverture centrale (138).

13. Ensemble de poignée de seringue selon la revendication 12,
dans lequel chacune parmi la paire de parties de préhension opposées est généralement concave le long d'un axe longitudinal du composant de poignée inférieur (112) et généralement convexe dans une direction perpendiculaire à l'axe longitudinal (140).

14. Ensemble de poignée de seringue selon la revendication 1,
dans lequel la pluralité de nervures (128) sont séparées par une pluralité d'évidements (132).
